## Europäisches Patentamt
## European Patent Office
## Office européen des brevets

(11) Veröffentlichungsnummer: **0 287 076 B1**

(12) # EUROPÄISCHE PATENTSCHRIFT

(45) Veröffentlichungstag der Patentschrift: **24.03.93**

(21) Anmeldenummer: **88105897.8**

(22) Anmeldetag: **13.04.88**

(51) Int. Cl.5: **C12P  21/00**, C12P 21/08, A61K 37/02, A61K 39/395, G01N 33/569

(54) **Rezeptor der Kleinen Rhinovirus Rezeptor Gruppe.**

(30) Priorität: **14.04.87 DE 3712678**

(43) Veröffentlichungstag der Anmeldung:
**19.10.88 Patentblatt  88/42**

(45) Bekanntmachung des Hinweises auf die
Patenterteilung:
**24.03.93 Patentblatt  93/12**

(84) Benannte Vertragsstaaten:
**AT BE CH DE ES FR GB GR IT LI LU NL SE**

(56) Entgegenhaltungen:
**EP-A- 0 169 146**

**CHEMICAL ABSTRACTS, Band 101, Nr. 11, 10. September 1984, Seite 332, Zusammenfassung Nr. 87106q, Columbus, Ohio, US; G. ABRAHAM et al.: "Many rhinovirus serotypes share the same cellular receptor", & J. VIROL. 1984, 51(2) 340-5**

(73) Patentinhaber: **BOEHRINGER INGELHEIM INTERNATIONAL GmbH**
**Postfach 20**
**W-6507 Ingelheim am Rhein(DE)**

(72) Erfinder: **Blaas, Dieter, Dipl.-Ing. Dr.**
**Liechtensteinstrasse 119/13**
**A-1090 Wien(AT)**
Erfinder: **Kuechler, Ernst, Prof. Dr.**
**Laudongasse 25/18**
**A-1080 Wien(AT)**
Erfinder: **Mischak, Harald, Dipl.-Ing. Dr.**
**Andreas Gruberstrasse 4**
**A-3100 St. Pölten(AT)**
Erfinder: **Neubauer, Christoph**
**Himmelstrasse 81**
**A-1190 Wien(AT)**

CHEMICAL ABSTRACTS, Band 104, Nr. 23, 9. Juni 1986, Seite 530, Zusammenfassung Nr. 204798s, Columbus, Ohio, US; J.E. TOMASSINI et al.: "Isolation of a receptor protein involved in attachment of human rhinoviruses", & J. VIROL. 1986, 58(2), 290-5

CHEMICAL ABSTRACTS, Band 108, Nr. 23, 6. Juni 1988, Seite 386, Zusammenfassung Nr. 201617d, Columbus, Ohio, US; H. MISCHAK et al.: "Characteristics of the minor group receptor of human rhinoviruses", & VIROLOGY 1988, 163(1), 19-25

**Beschreibung**

Die vorliegende Erfindung betrifft den Rezeptor der kleinen Rezeptorgruppe von humanen Rhinoviren, seine Reinigung, sowie dessen Verwendung.

Humane Rhinoviren repräsentieren eine große Gattung innerhalb der Familie der Picorna-Viren und beinhalten über 90 verschiedene Serotypen (6,11) Diese RNA-Viren befallen den Respirationstrakt des Menschen und verursachen akute Infektionen, die zu Schnupfen, Husten, Heiserkeit etc. führen, und allgemein als Erkältung bezeichnet werden (15). Infektionen durch Rhinoviren zählen zu den häufigsten Erkrankungen des Menschen. Obwohl der Verlauf der Erkrankungen meist harmloser Natur ist, führen Erkältungen doch zu einer allgemeinen Schwächung des Organismus. Dadurch kann es zu Sekundärinfektionen durch andere Pathogene kommen.

Die große Gruppe der humanen Rhinoviren kann in zwei Untergruppen geteilt werden, wenn als Kriterium der Zuordnung die Kompetition um Bindungsstellen an der Zelloberfläche humaner Zellkulturzellen (in der Regel HeLa Zellen) herangezogen wird. Diese ursprüngliche Zuordnung einiger weniger Vertreter der Rhinoviren (10) konnte durch breit angelegte Experimente auf 88 Vertreter ausgeweitet werden (4,1). Das Ergebnis dieser Experimente legt den Schluß nahe, daß trotz der großen Zahl überraschenderweise nur zwei voneinander verschiedene Rezeptoren an der Zelloberfläche existieren, an die Vertreter der einen oder der anderen Rhinovirusgruppe binden können. Bisher konnten 78 Serotypen der großen "Rhinovirus-Rezeptor-Gruppe", 8 der kleinen "Rhinovirus-Rezeptor-Gruppe" zugeordnet werden (RVRG). 2 weitere Vertreter verhielten sich nicht eindeutig, so daß keine endgültige Einteilung möglich war (1).

Abraham, G. und Colonno, R.J., (1984), J. Virol. 51 340-345) stellten mit Hilfe von Kompetitions-Bindungs-Assays fest, daß wahrscheinlich zwei Rezeptorfamilien für die Aufnahme humaner Rhinoviren existieren, wobei der Großteil der Rhinoviren an einen Rezeptor bindet. Dabei können Rhinoviren einer Rezeptorgruppe die Bindung zu dem Rezeptor gegenseitig kompetitiv inhibieren. Diese Kompetitionsexperimente erlauben jedoch keine Rückschlüsse auf die Beschaffenheit der Rezeptoren.

In den letzten Jahren ist eine beträchtliche Zunahme an Rhinovirusinfektionen in Ballungszentren festgestellt worden. Während es bei den meisten anderen Infektionskrankheiten zu einer langdauernden oder bleibenden Immunität gegen den betreffenden Krankheitserreger kommt, können Infektionen durch Rhinoviren immer wieder auftreten. Der Grund für das Fehlen einer bleibenden Immunität ist die große Vielfalt an Rhinovirusstämmen, die untereinander keine oder nur wenig immunologische Kreuzreaktion zeigen (6, 11). Nach erfolgter Infektion lassen sich zwar Antikörper gegen den betreffenden Virusstamm nachweisen, doch gewähren diese keinen Schutz gegen andere Rhinovirusstämme. Auf Grund der großen Anzahl von Stämmen, die in der Bevölkerung zirkulieren, sind wiederholte Infektionen durch Rhinoviren möglich.

Daher bietet das Vorhandensein von nur zwei Rezeptoren vielversprechende Ansatzmöglichkeiten für eine erfolgreiche Bekämpfung rhinoviraler Infektionen.

Da Rezeptoren im allgemeinen hochspezifisch sind, besteht die Möglichkeit, mit geeigneten Substanzen gezielt eine Beeinflussung der Rezeptoren, beispielsweise durch Blockieren der Rezeptoren zu erreichen. Werden den Rezeptor blockierende Substanzen eingesetzt, kann das Eindringen rezeptorspezifischer Viren in die Zelle verhindert werden. Dieselben Substanzen, die auf diesem Weg eine Infektion verhindern können, sind auch zur Therapie einer manifesten Rhinovirusinfektion verwendbar. Die Schaffung solcher Substanzen wird wesentlich erleichtert, in manchen Fällen gar erst gezielt möglich, wenn der entsprechende Rezeptor charakterisiert ist.

Eine Aufgabe der vorliegenden Erfindung war es daher, den Rezeptor für die kleine RVRG zu isolieren und zu reinigen.

Die einzigen bisher verfügbaren Angaben zu rhinoviralen Rezeptoren beziehen sich auf den Rezeptor der großen RVRG.

Die Reinigung und Charakterisierung des Rezeptors der großen RVRG wurde mit Hilfe eines monoklonalen Antikörpers durchgeführt, der durch Immunisierung von Mäusen mit HeLa Zellen erhalten worden war. Dieser Rezeptor ist glykosyliert und hat ein Molekulargewicht um 440 kD in nativem Zustand, Denaturierung mit Natrium Laurylsulfat führt zu einer Dissoziation in Untereinheiten mit 90 kD, was den Schluß nahelegt, daß der funktionelle Rezeptor als Pentamer vorliegt (17). Der Rezeptor für die kleine RVRG wurde bisher weder charakterisiert noch gereinigt. Die einzigen Daten über diesen Rezeptor weisen auf seinen Aufbau aus Protein hin und zeigen überdies, daß dieser - oder ähnliche-Proteine auch auf Zellen einer Reihe anderer Spezies vorhanden sind. Dies unterscheidet den Rezeptor der kleinen RVRG wesentlich vom Rezeptor der großen Gruppe, der nur auf menschlichen und in wenigen Fällen auf Affenzellen gefunden werden konnte. Eine Beeinflussung, beispielsweise eine Blockierung des Rezeptors durch Substanzen, die das Eindringen des Virus in die Zelle verhindern, erscheint als mögliche Vorbeugung oder auch als Therapie einer bereits vorhandenen Infektion geeignet.

Aufgabe der vorliegenden Erfindung war es daher, die Voraussetzungen zur Herstellung von Substanzen zu schaffen, die einen Schutz gegen Infektionen durch Rhinoviren der kleinen Rezeptorgruppe ermöglichen.

Gelöst wurde die Aufgabe in der vorliegenden Erfindung dadurch, daß der Rezeptor aus Zellmembranen, beispielsweise aus HeLa-Zellmembranen isoliert wurde. Diese Zellen wurden nach an sich bekannten Verfahren in Suspension kultiviert, die Zellen aufgebrochen, Zellkerne entfernt und die Membranen gereinigt. Die in den Membranen befindlichen Rezeptoren wurden anschließend solubilisiert. Zur Optimierung der Solubilisierung aktiver Rezeptoren aus gereinigten HeLa-Zellen wurden verschiedene Detergentien bei verschiedenen Konzentrationen ausgetestet. Maßgeblich für die Wahl eines bestimmten Detergens war die Fähigkeit, möglichst viel Membranmaterial mit möglichst hoher Virus-Bindungs-Aktivität zu solubilisieren. Als am besten geeignet erwies sich 1% 1-O-n-Octyl-$\beta$-D-glucopyranosid (Fig. 0).

Die unlöslichen Bestandteile wurden entfernt und die in Lösung befindlichen Rezeptoren weiter gereinigt. Um die Virusbindungsaktivität verfolgen zu können, wurde ein sensitiver Filterbindungstest entwickelt, der die Bindung $^{35}$S-markierten Virus an Rezeptoren ermöglicht, die auf Nitrozellulose Papier immobilisiert worden waren.

Die für den Test notwendigen Viren wurden in an sich bekannter Weise kultiviert und gereinigt(13).

Die Reinigung der erfindungsgemäßen Rezeptoren erfolgte mittels chromatographischer Methoden.

Da bekannt ist, daß die meisten Membranproteine glykosyliert sind, wurde der Rezeptor an einer Lens culinaris Lectin Säule gereinigt. Dieses Lectin besitzt Spezifität zu $\alpha$-D-Glukose und $\alpha$-D-Mannose Einheiten (16). Eluiert wurde gebundenes Material mit 1M $\alpha$-D-Methylglucosid in phosphatgepufferter NaCl-Lösung mit 1% Octylglucosid.

Aliquots wurden auf Nitrozellulose in Duplikat aufgetragen und mit sowohl nativem als auch erhitztem Virus inkubiert. Die Autoradiographie der Fraktionen zeigte starke Bindung zum nativen Virus bei dem Material, das eluiert worden war, verglichen zu den Fraktionen aus dem Durchlauf. Schwache Bindung zum Durchlaufmaterial zeigte der erhitzte Virus. Dies deutet auf unspezifische Interaktionen, die durch den hohen Anteil hydrophober Proteine hervorgerufen werden; erhitzter Rhinovirus weist erhöhte Hydrophobizität auf (9). Da festgestellt worden war, daß sich gereinigter Virus bei längerer Lagerung bei 4°C allmählich in Partikel umwandelt, die die gleiche Antigenizität aufweisen wie erhitzter Virus (C-Determinanten), wurden diese Kontaminationen durch Immunopräzipitation mit C-Determinanten spezifischen monoklonalen Antikörper, beispielsweise mAK 2G2, unmittelbar vor Durchführung des Bindungstests abgetrennt. Diese monoklonalen Antikörper erhält man in an sich bekannter Weise durch Immunisierung von Mäusen oder Kaninchen mit C-Determinanten und anschließender Klonierung nach Köhler und Milstein (18).

Neben der L. culinaris Lectin Säule wurden auch Concanavalin A-, Ricin- und Heparin-Sepharose-Säulen verwendet, um den Rezeptor zu reinigen. Durchfluß und eluiertes Material wurden wie oben getestet. Con.A Sepharose Säulen wurden mit 1 M $\alpha$-D-Methylmannosid eluiert, wobei annähernd 20% Bindungsaktivität wiedergewonnen werden konnte; Elution der Ricin-Säule mit 1 M Galaktose führte zu annähernd 100% wiedergewinnbarer Bindungsaktivität. Im Gegensatz zum Rezeptor für die Coxsackie B Virus Gruppe (7) hielt Heparin-Sepharose die Bindungsaktivitat nicht zurück.

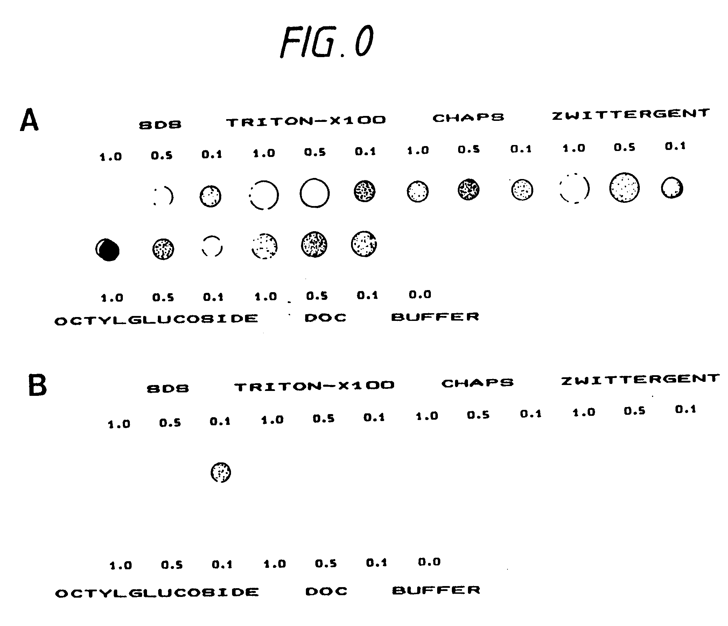

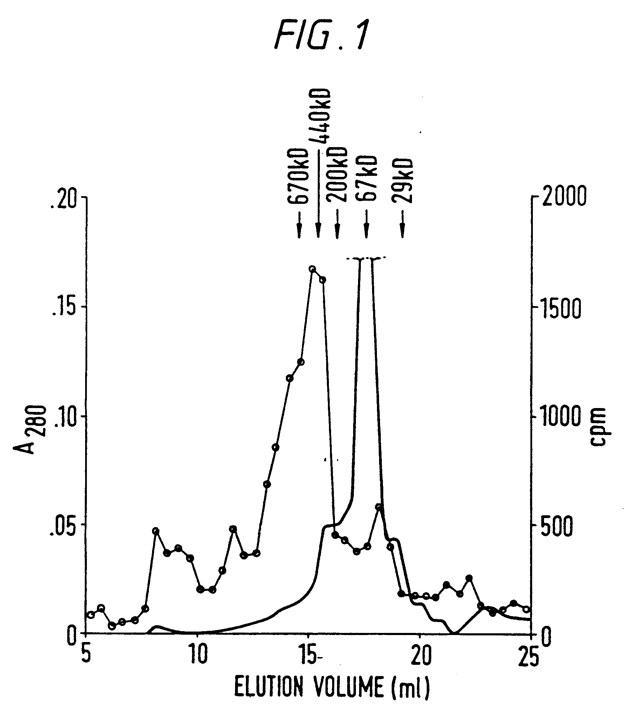

Das Eluat der L. culinaris Säule wurde auf einer Superose Säule mit Hilfe der FPLC (Pharmacia) aufgetrennt (Gelpermeationschromatographie). Durch Vergleich mit Markerproteinen konnte das Molekulargewicht des aktiven Rezeptors zu 450 kD bestimmt werden. Gleichzeitig konnte ein Großteil von kontaminierenden Proteinen abgetrennt werden (Figur 1).

Der Rezeptor der Kleinen Rhinovirus Rezeptor Gruppe wandert in einem Polyacrylamidgel in Gegenwart von SDS mit einem scheinbaren Molekulargewicht von 120 kD. In einigen Fällen kann auch eine kaum sichtbare Bande mit einem geringfügig niedrigeren Molekulargewicht beobachtet werden, die möglicherweise eine Modifikation der Hauptmenge des Rezeptorproteins dargestellt (Fig. 5, Spalte 1). Die Molekulargewichte beider Formen des Rezeptors sind beträchtlich höher als das für den Großen Rhinovirus Rezeptor gefundene (90 kD).

Da die Rezeptorproteine beider Gruppen ein Molekulargewicht von ca. 450 kD in ihrer nativen Form aufweisen, ist es nicht unwahrscheinlich, daß ihr Aufbau aus Untereinheiten ähnlich ist. Das tatsächliche Molekulargewicht mag jedoch von dem durch Gelpermeationchromatographie bestimmten abweichen, bedingt durch die geringe Differenz der Retentionsvolumina von Proteinen in diesem hohen Molekulargewichtsbereich. Die Picornavirusstruktur zeigt eine tiefe Furche (den Canyon), die um die fünffachen Achsen der ikosahedralen Symmetrie verläuft und die möglicherweise die Rezeptorbindungsstelle enthält (19). Es wird angenommen, daß der Rezeptor der Großen Rhinovirus Rezeptor Gruppe und der Rezeptor für die Coxsackie B Virus Gruppe, die die Viren über die fünffachen Achsen binden (20). Die Frage ob der Rezeptor der Kleinen Gruppe ein Pentamer ist, bleibt unbeantwortet, da das Molekulargewicht seiner Untereinheiten sehr hoch ist im Vergleich zum Rezeptor der Großen Gruppe.

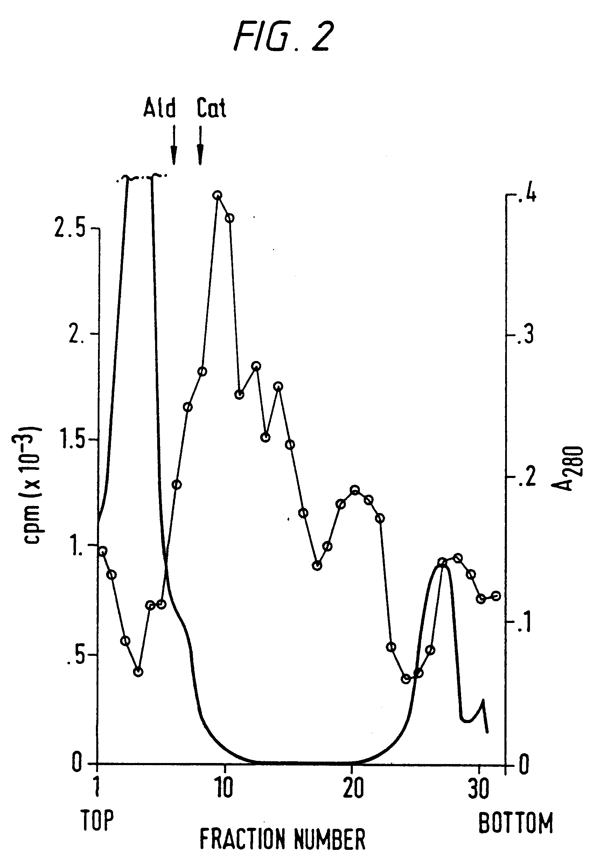

Mit Hilfe einer Zuckergradientenzentrifugation war es möglich die Sedimentationskonstante des Rezeptors zu bestimmen. Hierzu wurde L. culinaris gereinigter Rezeptor auf einen Zuckergradienten aufgetragen und zentrifugiert. Das Aktivitätsmaximum wurde an der Position des Gradienten gefunden, der der Sedimentationskonstante von 28,4 S entspricht (Figur 2).

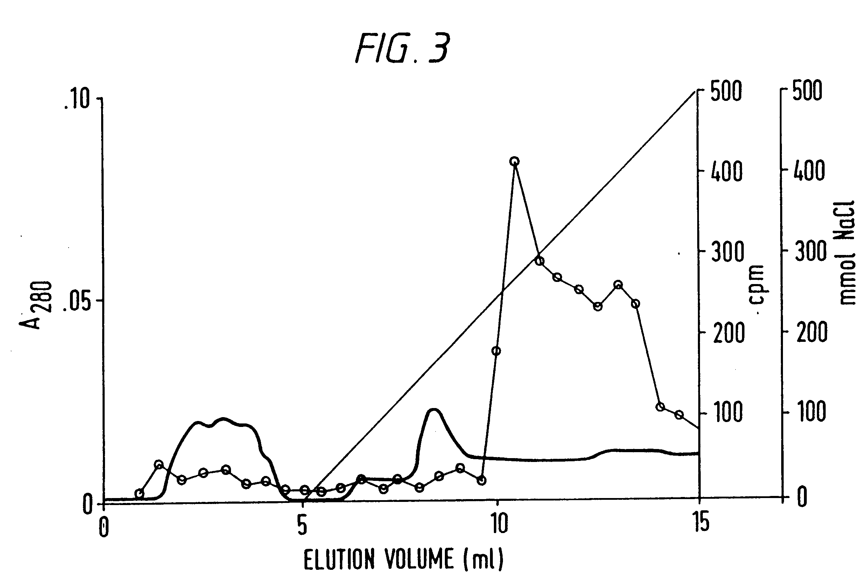

In Vorversuchen war festgestellt worden, daß der Rezeptor von einer Anionenaustauschchromatographie Säule nicht mehr eluiert werden konnte. Da der Rezeptor unempfindlich gegenüber Neuraminidase ist, wurde die Sialinsäure von dem Glycoprotein entfernt, um die ionische Wechselwirkung mit dem Säulenmaterial zu vermindern. Die Probe wurde dann auf eine Mono Q Säule (Pharmacia) aufgetragen, der Rezeptor mit einem NaCl-Gradienten eluiert. Die Bindungsaktivität konnte als breiter Peak bei etwa 250 mM NaCl nachgewiesen werden (Figur 3).

Die Reinigung der erfindungsgemäßen Rezeptoren ist auch möglich durch eine Kombination der chromatographischen Reinigungsschritte an verschiedenen Chromatographiematerialien.

Die chemischen Eigenschaften und die strukturellen Erfordernisse des erfindungsgemäßen Rezeptors für die virale Bindung wurden mit Hilfe von Enzymen und chemischen Reagenzien ermittelt (Tabelle 1).

Trypsin Behandlung zerstört die Bindungsaktivität vollständig. Dies stimmt überein mit bereits bekannten Resultaten aus enzymatischer Behandlung von Zell-Oberflächen (14) und deutet auf den Proteincharakter des Rezeptors hin.

Behandlung des solubilisierten Rezeptors mit Neuraminidase resultierte wiederholbar in einem leichten Anstieg der Bindungsaktivität. Diese Behandlung führt möglicherweise zu einer besseren Zugänglichkeit der Region auf dem Rezeptormolekül, die das Ziel der Virus-Interaktion darstellt. Hieraus ergibt sich, daß Sialinsäure nicht notwendig ist für die Virus Bindung.

Dithiotreitol (DTT) zerstört die Bindungsaktivität, was auf die Beteiligung von Disulfidbrücken bei der Aufrechterhaltung der korrekten Faltung des Proteins schließen läßt. Das überraschend hohe Molekulargewicht, ermittelt durch Gelpermeationschromatographie und Gradientenzentrifugation, deutet auf eine oligomere Struktur des Rezeptor Moleküls. Die Sensitivität gegenüber DTT konnte darauf schließen lassen, daß intermolekulare Disulfidbrücken erforderlich sind für die Assoziation der hypothetischen Untereinheiten.

Behandlung mit Iodacetamid reduziert nur wenig die Bindungsaktivität und deutet darauf hin, daß freie Sulfhydryl-Gruppen nicht erforderlich sind für eine effiziente Bindung.

In Gegenwart von Ethylendiaminotetraessigsäure (EDTA) bei der Inkubation der Nitrozellulosefilter mit $^{35}$S-markiertem Virus konnte keine Bindung festgestellt werden. Dies stimmt überein mit früheren Untersuchungen, die die Notwendigkeit der Gegenwart divalenter Kationen für die Interaktion der Rhinoviren mit der Zelloberfläche zeigten (12).

Kompetitions-Bindungs-Assays zwischen Serotyppaaren waren verwendet worden um die humanen Rhinoviren in die zwei Rezeptorklassen einzuordnen (10,1). In der vorliegenden Erfindung wurden daher HRV2 und HRV49 als Vertreter der kleinen Rezeptorgruppe und HRV89 als Vertreter der großen Rezeptorgruppe bei Kompetitions-Experimenten eingesetzt, um die Spezifität der erfindungsgemäßen Rezeptoren herauszufinden. Die Nitrozellulosefilter mit immobilisierten Rezeptoren wurden in Gegenwart von einem ungefähr 20-fachen Überschuß von entweder HRV2 oder HRV89 mit markiertem HRV2 inkubiert. Wie in Tabelle II gezeigt, wurde die Bindung massiv unterdrückt in Gegenwart von nicht-markiertem HRV2, nicht jedoch beeinflußt von HRV89. Zur Absicherung dieser Ergebnisse wurden diese Versuche mit markiertem HRV49 wiederholt unter Verwendung von HRV2 und HRV89 als Kompetitoren. Wiederum ist es offensichtlich, daß HRV2 die Bindung massiv reduziert, HRV89 jedoch keinen Einfluß ausübt.

Obwohl HRV2 und HRV89 an verschiedene Rezeptoren binden, zeigen ihre Capsid-Proteine überraschend hohe Ähnlichkeit (5). Ein detaillierter Strukturvergleich zwischen HRV2 und HRV14, basierend auf der Röntgenstrukturanalyse des HRV14 wurde kürzlich angestellt (2). Beide, HRV14 und HRV89 binden an den Rezeptor der großen RVRG. Erwartet werden kann daher, daß an der hypothetischen Rezeptor Bindungsstelle ein Cluster an konservierten Aminosäuren zu finden sein wird. Bisher war es jedoch noch nicht möglich, ein einfaches Muster konservativer Aminosäuren innerhalb der Canyon-Region zu entdecken.

Durch die vorliegende Erfindung ist es erstmals möglich, Rezeptoren für die kleine RVRG in im wesentlichen reiner Form herzustellen.

Durch die erfindungsgemäßen Rezeptoren ist es erstmals moglich, die Virus/Rezeptor Wechselwirkungen gezielt zu untersuchen. Von besonderer Bedeutung hierbei ist die Lokalisierung der Bereiche auf den Rezeptoren, die für die virale Wirkung letztendlich verantwortlich sind. Bei Kenntnis dieser Bereiche sollte es möglich sein, Substanzen herzustellen, die spezifisch gegen diese Bereiche gerichtet sind, um so möglicherweise eine Blockierung der Rezeptoren für eine Vielzahl verschiedener Rhinoviren zu erzielen.

Gegenstand der vorliegenden Erfindung sind Rezeptoren, die nach dem beschriebenen Verfahren herstellbar sind und Vertreter der kleinen RVRG binden.

Gegenstand der vorliegenden Erfindung sind auch die Rezeptoren, die durch, dem Fachmann bekannte Verfahren aus den natürlichen Rezeptoren herstellbar sind. Beispielhaft genannt seien hier die durch Behandlung mit Reduktionsmitteln erhältlichen Untereinheiten des natürlichen Rezeptors, die sich beispielsweise durch elektrophoretische Verfahren reinigen lassen. Verwendbar sind diese Untereinheiten beispielsweise zur Herstellung von polyclonalen und/oder monoklonalen Antikörpern, die präparativ, diagnostisch und/oder therapeutisch in ähnlicher Weise einsetzbar sind wie die entsprechenden Antikörper gegen die natürlichen Rezeptoren. Auch die Rezeptoruntereinheiten sind in ähnlicher Weise wie die natürlichen Rezeptoren einsetzbar.

Gegenstand der vorliegenden Erfindung sind auch die Modifikationen der natürlichen Rezeptoren und/oder deren Untereinheiten, die durch gezielte enzymatische Behandlung erhältlich sind. Wie in der vorliegenden Erfindung gezeigt wurde, zerstört die Behandlung mit Trypsin die Bindungsaktivität der erfindungsgemäßen Rezeptoren während Neuraminidase einen leichten Anstieg der Aktivität verursachte. Daher ist es denkbar und für den Fachmann auf nicht erfinderische Weise überprüfbar, daß bestimmte Enzyme und/oder chemische Reagenzien zu Rezeptoren führen, die entweder in ihrer Wirkung verbessert sind und/oder besser applizierbar und verwendbar sind und/oder verbesserte Stabilität aufweisen, verglichen mit den natürlichen Rezeptoren. Diese Modifikationen können beispielsweise dazu führen, daß an allen oder einzelnen Untereinheiten der natürlichen Rezeptoren Teile der Proteinkette abgetrennt, herausgeschnitten und/oder daß die Proteinketten aufgeschnitten werden.

Neben diesen Modifikationen ist es weiterhin möglich, die natürlichen Rezeptoren beispielsweise durch gesteuerte Reduktion entweder vollständig oder teilweise in die Untereinheiten zu überführen. Diese mehr oder weniger großen Untereinheiten lassen sich auch durch beispielsweise gezielte Oxidation zu mehr oder weniger großen Einheiten verknüpfen, die gegenüber den natürlichen Rezeptoren umgeordnet sind.

Zur Aufspaltung von Disulfidbrücken geeignete Reduktionsmittel sind beispielsweise Thiol-Verbindungen, wie Thiophenol, 4-Nitrothiophenol, 1,4-Butandithiol und insbesondere 1,4-Dithiothreitol. Die Reduktion wird vorteilhaft in einem wässrig-alkalischen Medium, beispielsweise in der verdünnten wässrigen Losung eines Alkalimetallhydroxids, z.B. Natriumhydroxid, Alkalimetallcarbonats, z.B. Natriumcarbonat oder einer organischen Base, insbesondere eines Triniederalkylamins, z.B. Triäthylamin, bei Raumtemperatur durchgeführt.

Oxidationsmittel, welche zur Neuknüpfung von Disulfidbindungen in den reduzierten Polypeptiden geeignet sind, sind beispielsweise Luftsauerstoff, welcher durch eine wässrige Lösung des Polypeptids, der gegebenenfalls eine katalytische Menge eines Übergangsmetallsalzes, z.B. Eisen-(III)-sulfat, Eisen-(III)-chlorid oder Kupfer-(II)-sulfat, beigefügt wurde, geleitet wird; Jod, auch in Form des Kaliumjodid-Adduktes $KJ_3$ welches vorzugsweise in alkoholischer, z.B. methanolischer, oder wässrig-alkoholischer, z.B. wässrig-methanolischer Lösung eingesetzt wird; Kaliumhexacyanoferrat-(III) in wässriger Lösung; 1,2-Dijodäthan oder Azodicarbonsäuredimethylester oder -diäthylester, welche in Wasser oder in einer Mischung bestehend aus Wasser und einem mit Wasser mischbaren Alkohol, z.B. Methanol, zur Reaktion gebracht werden. Die Oxidation wird insbesondere bei Raumtemperatur ausgeführt.

Die Abtrennung der Reagentien, insbesondere der Salze und der Oxidations- bzw. der Reduktionsmittel und ihrer Folgeprodukte, von der gewünschten Verbindung erfolgt nach an sich bekannten Methoden, beispielsweise durch Molekulargewichtsfiltration, z.B. an Sephadex oder Biogel.

Sämtliche Modifikationen lassen sich in ähnlicher Weise wie die natürlichen erfindungsgemäßen Rezeptoren verwenden. Die hierdurch erhältlichen Produkte wie beispielsweise die Antikörper gehören ebenso wie die Modifikationen zum Umfang der vorliegenden Erfindung.

Der erfindungsgemäße Rezeptor ist löslich, so daß er leicht gehandhabt und verwendet werden kann.

Es ist aber auch möglich, die Rezeptoren an einen festen Träger zu binden und in dieser Form für diagnostische und prapärative Zwecke einzusetzen. Als Träger kommen alle üblichen festen Trager wie Polystrol, Glas, Dextrane, aber auch biologische Membranen und Lipid-Vesikel in Frage.

Weiterhin ist es möglich, an die Rezeptoren übliche Marker zu binden und in dieser Form diagnostisch einzusetzen. Weiterhin ist es möglich, die Rezeptoren zur therapeutischen Behandlung von viralen Infektionen einzusetzen.

Sofern die erfindungsgemäßen Rezeptoren an einen Träger gebunden werden, können sie sowohl diagnostisch als auch präparativ zur Bindung des viralen Proteins verwendet werden, beispielsweise mittels der sogenannten Affinitätschromatographie. Diagnostisch kann ein virales Protein durch einen an einen Träger gebundenen Rezeptor in üblicher Weise erfaßt werden, z.B. mittels Antikörpern oder markierten Antikörpern. Als Markierung kommt beispielsweise radioaktive Markierung, ein Enzym oder eine fluoreszierende Gruppe in Frage.

Bei der therapeutischen Verwendung der erfindungsgemäßen Rezeptoren können diese in entsprechend hoch gereinigter Form injiziert werden, so daß sie dann kompetitiv gegenüber dem natürlichen Rezeptor inhibieren. Vorzugsweise wird man für diesen Zweck lösliche Rezeptoren verwenden. Derartige Lösungen können auch zur Diagnostik und Differentialdiagnostik herangezogen werden.

Von außerordentlicher Bedeutung ist die Verwendung der erfindungsgemäßen Rezeptoren zur Erzeugung von polyclonalen und/oder monoclonalen Antikörpern, die spezifisch gegen die in den Zellmembranen befindlichen Rezeptoren wirken. Derartige Antikörper können zunächst wiederum diagnostisch eingesetzt werden zur Darstellung und Bestimmung der Rezeptoren auf Zellen oder zellbiologischem Material. Weiterhin können sie therapeutisch eingesetzt werden zum Blockieren der Rezeptoren in den Zellmembranen.

Sie eröffnen damit völlig neue Wege und Möglichkeiten.

## Legende zu den Abbildungen

Figur 0 — Solubilisierung der Rezeptoren mit verschiedenen Detergentien (A: nat. Virus, B: denat. Virus).

Figur 1 — Gelpermeationschromatographie des solubilisierten Rezeptors auf einer Superose 6 HR 10/30 Säule. Membranen äquivalent zu $10^8$ Zellen wurden in OG (15 mM Natriumphosphat pH 7,4, 150 mM NaCl, 1 mM $MgCl_2$, 1 mM $CaCl_2$ (PBS) mit 1% Octylglucosid) solubilisiert, auf eine 1 ml L. culinaris Säule aufgetragen und das adsorbierte Material mit 1 M $\alpha$-D-Methylglucosid in OG eluiert. Das Eluat wurde in einem Centricon Röhrchen auf 0,5 ml konzentriert und auf die Superose Säule aufgetragen. Die Säule wurde mit 0,2 ml/min OG entwickelt und Fraktionen von 0.5 ml wurden gesammelt. Die Bindungsaktivität der einzelnen Fraktionen, die Positionen der Markerproteine (in einem getrennten Experiment bestimmt) und die Extinktion bei 280 nm sind gezeigt.

Figur 2 — Zuckergradientenzentrifugation des solubilisierten Rezeptors. Material das von einer L. culinaris Säule eluiert worden war wurde konzentriert (siehe Legende zu Figur 1) und auf einem 10 - 40% Zuckergradienten in OG aufgetrennt. Die Bindungsaktivität der Fraktionen (0.4 ml), die Position der Markerproteine Katalase (Cat) und Aldolase (Ald) sowie die Extinktion bei 280 nm sind gezeigt.

Figur 3 — Mono Q Anionen Austausch Chromatographie. Fraktionen 14 bis 16 der Gelpermeationschromatographie (Figur 1) wurden mit Neuraminidase behandelt und das Material mittels FPLC auf Mono Q aufgetrennt. Die Bindungsaktivität der einzelnen Fraktionen (0,5 ml), die Extinktion bei 280 nm und der Verlauf des Gradienten sind gezeigt.

Figur 4 — Nachweis des Rezeptors auf Western Blots. Virusbindungsaktivität von der Mono P Anionentauschersäule wurde auf eine Superose 6 HR 10/30 Säule aufgetragen. Fraktionen von 1,2 ml wurden gesammelt. Die Proteine wurden als $A_{280}$ verfolgt, die Positionen von Proteinmarkern (Thyroglobulin, 670k; Apoferritin 440k; $\beta$-Amylase, 200k) sind auch gezeigt (a). Die Proteine in den Fraktionen von der Superosesäule wurden konzentriert und auf drei 6% SDS-Polyacrylamidgele aufgetragen. Ein Gel wurde mit Coomassie Blau gefärbt (b), die Proteine des zweiten und des dritten Gels wurden auf Nitrozellulosemembran übertragen (c, d). Der Blot wurde mit $^{35}$S markiertem HRV2 in Abwesenheit (c) oder in Gegenwart eines Überschusses unmarkierten HRV2s inkubiert (d); Die Positionen der Markerproteine (2-Macroglobulin, 180k; $\beta$-Galactosidase, 116k; Fructose-6-phosphatkinase, 84k) und der Rezeptorbande sind mit Pfeilen markiert.

Figur 5 — Autoradiographie von Western Blots, die von den vereinigten Fraktionen mit Virusbindungsaktivität von der Superosesäule erhalten wurden (siehe Fig. 4). Die Blots wurden mit $^{35}$S markiertem HRV2 inkubiert. Die Proben wurden vor dem Auftragen auf das Gel mit SDS bei Raumtemperatur inkubiert, Spur 1; die Probe wurde in SDS gekocht, Spur 2, die Probe wurde mit 10 mM Dithiothreitol inkubiert, Spur 3; ein mit Spur 1 identischer Blot wurde mit $^{35}$S markiertem HRV2 in Gegenwart von 10 mM EDTA inkubiert, Spur 4, oder wurde mit $^{35}$S markiertem HRV2 inkubiert, das 10 Minuten auf 56°C erhitzt worden war, Spur 5.

## Materialien

1-0-n-Octyl-8-D-glucopyranoside, Tween 40 und 3-(3-Cholamidopropyl)-dimethylammonio-1-propansulfonat (CHAPS) stammten von Sigma, N-Tetradecyl-N,N-dimethyl-ammonio-3-propansulfonat (Zwittergent 3 - 14) von Serva. Die anderen Detergentien stammten von Merck. Trypsin von Miles, $^{35}$S-Methionin (1350 Ci/mmol) von Amersham.

Beispiel 1:

Herstellung der Viren

HRV2, HRV49 und HRV89 wurden im wesentlichen wie beschrieben in HeLa-Zell-Suspension kultiviert und gereinigt (13). Beispielhaft ausgeführt sei hier die Kultivierung, Isolierung und Reinigung von HRV2.

HeLa-Zellen (Stamm HeLa-Ohio, 03-147, Flow Laboratories, England) wurden in Suspension bei 37°C gezüchtet. Das Suspensionsmedium (Thomas, D.C., Conant, R.M. und Hamparian, V.U., 1970, Proc. Soc. Exp. Biol. Med. 133, 62 - 65; Stott, E.J. und Heath, G.F., 1970, J. gen. Virol, 6, 15 - 24) bestand aus einer Joklik-Modifikation von MEM für Suspension (Gibco 072-1300) und 7% Pferdeserum (Seromed 0135). Die Inokulationsdichte betrug 5 - 10 x $10^4$ Zellen/ml, das Volumen 500 ml. Die Suspension wurde bei einer Zelldichte von 1 x $10^6$ Zellen/ml unter sterilen Bedingungen bei 300 g 10 min zentrifugiert. Der Überstand wurde abgesaugt und die Zellen in 100 ml Infektionsmedium (Joklik-Modifikation von MEM für Suspensionskultur mit 2% Pferdeserum und 2 mM $MgCl_2$) resuspendiert. Durch mehrmaliges vorsichtiges Aufsaugen in einer 20 ml Pipette wurden die Zellen homogen im Infektionsmedium verteilt. Danach wurde auf 500 ml aufgefüllt. Anschließend wurde die Zellsuspension auf 34°C gebracht und mit HRV2(zweimal Plaque - gereinigt) bei einer Multiplizität von 0,1 Viren/Zelle infiziert. Der HRV2Stamm wurde von der American Type Culture Collection (ATCC VR-482 und VR-1112) bezogen. Der verwendete Stamm wurde durch Antiserum gegen HRV2(American Type Culture Collection, Cat.No. ATCC VR-1112 AS/GP) neutralisiert.

Als Kontrollserum diente ein Antiserum gegen HRV7 (Cat. No. ATCC VR - 1117 AS/GP), das keine Neutralisation zeigte. Nach 60 Stunden bei 34°C wurde das Virus geerntet. Virus wurde sowohl aus den Zellen bzw. Zellfragmenten als auch aus dem Medium gewonnen.

Zu diesem Zweck wurde das Medium von infizierten Zellen und Zellfragmenten durch eine 10 min Zentrifugation bei 1500 g abgetrennt und abgesaugt. Der Niederschlag wurde bei -70°C eingefroren.

Die Zellniederschläge von 12 l Suspensionskultur wurden vereinigt, in 40 ml Tm-Puffer (20MM Tris/HCl, pH 7,5. 2mM $MgCl_2$) resuspendiert, 15 min auf Eis gestellt, anschließend im Dounce-Homogenisator aufgebrochen und das Gemisch 30 min bei 6000 g zentrifugiert. Der Niederschlag wurde anschließend noch einmal in 10 ml TM-Puffer gewaschen. Die beiden Überstände wurden vereinigt und 3 Stunden bei 110 000 g zentrifugiert, um das Virus zu pelletieren. Das Viruspellet wurde dann in 10 ml KTMP-Puffer (50 mM KCl, 50 mM Tris/HCl, pH 7,5, 5 mM $MgCl_2$, 2 mM Mercaptoethanol, 1 mM Puromycin, 0.5 mM GTP) aufgenommen und nach Zugabe von 150 $\mu$g DNase I (Sigma, Ribonukleasefrei) 1 Stunde auf Eis inkubiert.

Aus dem Infektionsmedium wurde das Virus unter Rühren bei 4°C mit Polyethylenglykol 6000 (PEG 6000; Merck) bei einer Konzentration von 7% und 450 mM NaCl gefällt (Korant, B.D., Lonberg-Holm, K., Noble, J. und Stasny, J.T., 1972, Virology 48, 71 - 86). Nach 4 Stunden in der Kälte wurde das Virus 30 min bei 1500 g abzentrifugiert, der Niederschlag in 10 ml KTMP-Puffer, der 75 $\mu$g DNase I enthielt resuspen-diert, das Gemisch 1 Stunde auf Eis inkubiert und anschließend bei -70°C eingefroren.

Die Virussuspension, die aus den Zellen und aus dem Medium gewonnen worden waren, wurden vereinigt, 5 min bei 37°C inkubiert, durch Zugabe von 60 ml kaltem TE-Puffer (10 mM Tris/HCl, PH 7,4, 1mM EDTA) abgekühlt und anschließend 5 min lang im Eisbad soniziert.

Danach wurde 30 min bei 6000 g zentrifugiert. Zum Überstand wurden 920 ml TE-Puffer, der 7% PEG 6000 und 450 mM NaCl enthielt, hinzugefügt, 4 Stunden vorsichtig bei 4°C gerührt und das entstandene Präzipitat 30 min bei 6000 g pelletiert. Der Niederschlag wurde abermals in 100 ml Tm-Puffer aufgenommen, das Virus wie oben durch Zugabe von PEG 6000 und NaCl gefällt und pelletiert. Der Niederschlag wurde in 40 ml TM-Puffer resuspendiert, die Suspension 30 min bei 6000 g zentrifugiert, und das Virus 3 Stunden bei 110 000 g pelletiert. Das Präzipitat wurde in 1 ml Tm-Puffer gelöst, nach Zugabe von 50 $\mu$g DNase I 1 Stunde bei 4°C inkubiert und anschließend 1 ml TE-Puffer hinzugefügt. Zur weiteren Reinigung wurde die Virussuspension auf Saccharosegradienten (10 - 30 % w/w in TE-Puffer) 4 Stunden bei 4°C bei 110 000 g zentrifugiert. Aus der Extinktion bei 260 nm wurden die das Virus enthaltenden Fraktionen ermittelt und mit TM-Puffer verdünnt, so daß die Endkonzentration an Saccharose 10% betrug. Danach wurde 8 Stunden bei 85 000 g zentrifugiert. Das Viruspellet wurde in 1 ml TM-Puffer aufgenommen und bei -70°C aufbewahrt. Zur Überprüfung der Reinheit der Viruspräparation wurde eine Elektrophorese auf einem 12,5%igem Polyacrylamid-Gel in Gegenwart von 0,1% Natriumdodecylsulfat durchgeführt (Laemmli, U.K., 1970 Nature (London) 277, 680-685) und die Proteinbanden mit Coomassie-Brillant-Blau angefärbt.

Beispiel 2:

Herstellung von $^{35}$S-Methionin markiertem Humanem Rhinovirus Serotyp 2 (HRV2)

2 HeLa-Zellmonolayer in 165 cm$^2$ Petrischalen wurden mit einer MOI (Multiplicity of Infection) von 40 mit HRV2 eine Stunde bei 34°C in methioninfreiem MEM medium (Gibco) mit 2% dialysiertem fötalem Kälberserum (Flow) infiziert. Die Zellen wurden 2 mal mit PBS gewaschen und die Inkubation bei 34°C in frischem Medium fortgesetzt. Nach 3 Stunden wurde jedem Monolayer 1 mCi $^{35}$S-Methionin (1350 Ci/mmol, Amersham) zugesetzt und für insgesamt 24 Stunden weiterinkubiert. Das Medium von infizierten Zellen und Zellfragmenten wurde durch eine 10 min Zentrifugation bei 1500 g abgetrennt und abgesaugt. Der Niederschlag wurde bei -70°C in 5 ml 10 mM Tris, 10 mM EDTA, pH 7,5 (Tris/EDTA) eingefroren und wieder aufgetaut. Überstand und gefroren/getauter Niederschlag wurden vereinigt und 30 min bei 45000 × g abzentrifugiert. Der Überstand dieser Zentrifugation wurde 2 Stunden bei 140000 × g zentrifugiert. Das Viruspellet wurde in 300 $\mu$l Tris/EDTA resuspendiert und das Virus über einen 10 -30% Saccharosegradienten wie oben gereinigt. Die einzelnen Fraktionen des Gradienten wurden mittels SDS-Elektrophorese in 12% Polyacrylamidgelen analysiert. Die reinen Virusfraktionen wurden vereint und in Gegenwart von 1 % BSA (bovine serum albumin) bei 4°C für maximal 4 Wochen gelagert.

Um aus den Präparationen aus Beispiel 1 und Beispiel 2 Virus mit geänderter Capsid-Struktur zu entfernen, wurden 20 $\mu$l Immunoadsorbans, das monoklonale Antikörper gegen die C-Determinante enthielt, beispielsweise mAK 2G2, mit dem gereinigten Virus 30 Min. inkubiert und pelletiert bevor die viralen Proben entfernt wurden.

Herstellung des Immunoadsorbans

Staphylococcus aureus (BRL) Zellen wurden zu einer 10% w/w Suspension in Wasser suspendiert. Die Zellen wurden zweimal mit PBS gewaschen, 1/5 Vol. Kaninchen-Anti-Maus IgG Serum (Behring) hinzugegeben; die Suspension wurde 1 h bei Raumtemperatur inkubiert. Die Zellen wurden zweimal mit PBS gewaschen und erneut 1 h mit 1/5 Vol. Maus-Ascites-Flüssigkeit, die monoklonale Antikörper gegen die C-Determinanten enthielt (z.B. mAK 2G2) inkubiert. Nach zweimaligem Waschen wurde pelletiert, das Pellet in PBS resuspendiert (10% W/W) und den Präparationen der radioaktiven Viren hinzugefügt.

Beispiel 3:

Solubilisierung des Rezeptors aus HeLa Zellen

HeLa-Zellen (Stamm HeLa-Ohio, 03-147, Flow Laboratories, England) wurden in Suspension bei 37°C gezüchtet. Das Suspensionsmedium (Thomas. D.C., Conant, R.M. und Hamparian, V.U., 1970, Proc. Soc. Exp. Biol. Med. 133, 62 - 65; Stott, E.J. und Heath, G.F., 1970, J. gen. Virol, 6, 15 - 24) bestand aus einer Joklik-Modifikation von MEM für Suspension (Gibco 072-1300) und 7% Pferdeserum (Seromed 0135). Die Inokulationsdichte betrug 5 - 10 × 10$^4$ Zellen/ml, das Volumen 500 ml. Die Suspension wurde bei einer Zelldichte von 1 × 10$^6$ Zellen/ml unter sterilen Bedingungen bei 300 g 10 min zentrifugiert. Der Überstand wurde abgesaugt und die Zellen 2 mal mit Phosphat gepufferter Kochsalzlösung (PBS) gewaschen. 10$^9$ Zellen wurden in 20 ml isotonischem Puffer (10 mM HEPES-KOH, pH 7.9, 140 mM KCL, 1.5 mM MgCl$_2$, 0.5 mM EDTA, 0.2 mM Phenylmethylsulfonylfluorid) suspendiert und in der Kälte mit 200 Stössen eines 50 ml Dounce Homogenisators aufgebrochen. Zellkerne wurden durch eine Zentrifugation für 3 Minuten bei 1000 × g abzentrifugiert. Die Membranen wurden dann mit Hilfe der Zwei-Phasen Methode (3) weiter gereinigt. Membranen entsprechend 2×10$^8$ Zellen pro ml wurden in PBS aufgenommen und in flüssigem Stickstoff gelagert. Zur Solubilisierung wurden 2 × 10$^8$ Zelläquivalente in 1 ml 1% Octylglukosid in PBS (OG) suspendiert und unlösliches Material durch eine Zentrifugation bei 80 000 × g für eine Stunde abgetrennt. Der Überstand wurde für die Säulenchromatographie verwendet.

Beispiel 4:

Filterbindungstest.

Auf Aktivität zu testende Fraktionen der Säulenchromatographien wurden in einem Dot-blot Apparat (Bio-Rad) auf eine Nitrozellulose Membran (BA85, Schleicher und Schüll) aufgetragen. Die Proben wurden bei Zimmertemperatur einsickern gelassen. Dann wurden Flüssigkeitsreste unter leichtem Wasserstrahl-Vakuum abgesaugt und unspezifische Protein Bindungsstellen mit 2% Rinderserumalbumin (BSA) in PBS bei 4°C über Nacht abgesättigt. Die Filter wurden anschließend mit $10^5$ cpm $^{35}$S- Methionin markiertem HRV2 in 1% Tween 40, 0.5% Natrium desoxycholat und 10 mM (3-(3-Cholamidopropyl)-dimethylammonio 1-propansulfonat) in PBS während einer Stunde inkubiert. Die Membranen wurden 2 mal mit 2% BSA in PBS gewaschen, getrocknet und die den Proben entsprechenden runden Areale ausgestanzt; die Radioaktivität wurde in einem Flüssigkeitsszitillationszähler gemessen.

Als Spezifitätskontrolle wurde HRV2 vor der Inkubation der Nitrozellulosefilter 10 Min. lang auf 56°C erhitzt (8). Nach dieser Behandlung konnte keine Bindung zu einer der Proben mehr festgestellt werden (Fig. 1 B). Hieraus wurde abgeleitet, daß die Bindung nativen HRV2 zum immobilisierten Material tatsächlich auf eine spezifische Interaktion des Virus mit dem Rezeptor zurückzuführen ist.

### Beispiel 5:

Affinititätschromatographie auf Lens culinaris Lectin Säulen.

$10^8$ Zelläquivalente wurden wie beschrieben solubilisiert und auf eine L. culinaris Säule (1 ml) aufgetragen, die mit OG equilibiert war. Die Säule wurde mit 5 ml OG gewaschen und gebundenes Material mit 2 ml 1 M $\alpha$-D-Methylglucosid in OG eluiert. Der Bindungstest zeigte, daß nahezu 100% der Bindungsaktivität wiedergewonnen werden konnten, wogegen etwa 90% des Gesamtproteins entfernt worden war.

### Beispiel 6:

Gelpermeationschromatographie

Das Eluat der L. culinaris Säule wurde mit einem Centricon Röhrchen (Ausschluß 30 kD) auf 0.5 ml konzentriert und auf einer Superose 6 HR 10/30 Säule (mit OG-Puffer äquilibiert) mit Hilfe der FPLC (Pharmacia) aufgetrennt. Durch Vergleich mit Markerproteinen konnte das Molekulargewicht des aktiven Rezeptors zu 450 kD bestimmt werden. Gleichzeitig konnte ein Großteil von kontaminierenden Proteinen abgetrennt werden (Figur 1).

### Beispiel 7:

Zuckergradientenzentrifugation

L. culinaris gereinigter Rezeptor (wie oben) wurde auf einen Zuckergradienten (10 - 40% in OG) aufgetragen und 8 Stunden bei 38 krpm bei 4°C zentrifugiert. Das Aktivitätsmaximum wurde an der Position des Gradienten gefunden, der der Sedimentationskonstante von 28,4 S entspricht. Die Positionen der Markerproteine wurden in einem anderen Gradienten bestimmt (Figur 2) Aufgrund der Gegenwart von Detergentien sedimentierten die Marker bei kalkulierten Sedimentationskoeffizienten von 15,0 S und 21.9 S (7,3 S und 11,3 S in Abwesenheit von Detergentien).

### Beispiel 8:

Anionen-Austauschchromatographie.

In Vorversuchen war festgestellt worden, daß der Rezeptor von Mono Q HR 5/5 Säulen (Pharmacia) nicht mehr eluiert werden konnte. Deshalb wurde über L. culinaris und Gelpermeation vorgereinigter Rezeptor mit 1 U Neuraminidase/mg Protein 60 Min. bei 37°C behandelt um die stark sauren Neuraminsäuregruppen zu entfernen. Die Probe wurde dann mit 10 mM Natriumphosphatpuffer pH 7, 1% Octylglucosid auf das 2 fache verdünnt und auf eine Mono Q Säule aufgetragen. Die Säule wurde mit einem Gradienten von 0 bis 1 M NaCl in demselben Puffer entwickelt. Die Bindungsaktivität konnte als breiter Peak bei etwa 250 mM NaCl nachgewiesen werden (Figur 3).

## Beispiel 9

### Isolierung und Reinigung des Rezeptors

Plasmamembranen von $2 \times 10^9$ HeLa Zellen wurden in 5 ml PBS, das 1% w/v 1-0-n-octyl-$\beta$-D-Glucopyranosid und 0,01% w/v je L-$\alpha$-p-Tosyl-L-Lysinchlormethylketon (TLCK), L-1-Tosylamid-2-phenylethylchlormethylketon (TPCK) und Phenylmethyl-sulphonylfluorid (PMSF) (alle von SIGMA) enthielt, innerhalb 10 Min. bei Raumtemperatur gelöst. Unlösliches Material wurde 30 Minuten bei 30 krpm im Beckmann 65 Festwinkelrotor abzentrifugiert. Der Überstand wurde auf eine 25 ml L. culinaris Lectin- säule, die mit OG (PBS, 1% w/v Octylglucosid) equilibriert worden war, aufgetragen und das gebundene Material mit 5 ml OG, das 1 M $\alpha$-Methylglucosid enthielt, eluiert. Das Eluat wurde zu 50% mit einem gleichen Volumen gesättigter Ammoniumsulfatlösung gesättigt. Das präzipitierte Material wurde in 2 ml Puffer A (10 mM TRIS-HCl (pH 7,5), 5 mM EDTA, 1% w/v Octyl-glucosid) gelöst, und in eine Mono P Anionenaustauschersäule, die an ein FPLC-System (Pharmacia) angeschlossen war, eingespritzt.

Die Proteine wurden mit Hilfe eines Gradienten von 0 bis 100% Puffer B (wie Puffer A jedoch 1,5 M NaCl enthaltend) getrennt. Die Fraktionen, die Virusbindungsaktivität enthielten, wurden vereinigt, mit einem Centriconröhrchen auf 0,5 ml konzentriert und auf einer Superose 6 Säule, die mit OG, das 5 mM EDTA enthielt, equilibriert worden war, aufgetrennt. Die Proteinkonzentration wurde als Extinktion bei 280 nm verfolgt (Fig. 4a).

Die Fraktionen von dieser Säule wurden auf 50 $\mu$l konzentriert, auf 0,1% w/v SDS gebracht und Aliquote auf drei 6% Polyacrylamidgelen, die 5 mM EDTA enthielten, getrennt (21). Die aufgetrennten Proteine im Gel wurden dann entweder mit Coomassie Blau angefärbt (Fig. 4b) oder elektrophoretisch auf eine Nitrozellulosemembran (22) übertragen, die mit $4 \times 10^5$ cpm $^{35}$S-markiertem HRV2 inkubiert worden war (s. oben). Die Nitrozellulose wurde getrocknet und autoradiographiert (Fig. 4c). Als Kontrolle für eine spezifische Bindung wurde ein identisch hergestelltes Nitrozellulosereplica in Gegenwart eines 20-fachen Überschusses unmarkierten HRV2's inkubiert (Fig. 4d). Festgestellt wurde, daß die Fraktionen 6 und 7 von der Superosesäule Material enthielten, das in der Lage war, HRV2 zu binden, wenn es auf Nitrozellulose übetragen worden war.

Die Autoradiographie zeigt einige Banden mit einem scheinbaren Molekulargewicht größer als 300 kD zusätzlich zu einer Position, die etwa 120 kD entspricht, verglichen mit Markerproteinen, die auf demselben Gel entwickelt wurden (Fig. 4c). Nur diese 120 kD Bande verschwindet in der Kontrolle, die überschüsssiges nicht-markiertes Virus enthält (Fig. 4d) und zeigt damit spezifische Wechselwirkung dieses Proteins mit HRV2 an. Das Polyacrylamidgel, das identische Proben enthält und mit Coomassie Blau angefärbt worden war, zeigte eine sehr schwache Bande an einer Position, die der radioaktiven Bande auf dem Western-Blot entspricht. Diese Bande findet man ausschließlich in den Proben von den Fraktionen 6 und 7 die Virusbindungsaktivität zeigten (Fig. 4 b).

## Beispiel 10

### Bindungstests

Die Wiederherstellung eines aktiven Rezeptors hängt von milden Bedingungen ab; Kochen in SDS beispielsweise zerstört die Aktivität irrevesibel (vgl. Fig. 5. Spalte 1 und 2). Wurde die Rezeptorpräparation mit 10 mM Diothiothreitol vor dem Aufbringen auf das Gel inkubiert, konnte keine Bindung beobachtet werden (Fig. 5, Spalte 3). Da die spezifischen Wechselwirkungen der Rhinoviren mit ihren Rezeptoren von der Anwesenheit divalenter Kationen abhängt (12) wurde der Blot einer Probe, die mit der auf Spur 1 aufgetragenen identisch war (Fig. 5), mit Virus in Gegenwart von EDTA inkubiert. Unter diesen Bedingungen konnte keine Bindung beobachtet werden (Fig. 5, Spalte 4).

Als weiterer Test wurde die Inkubation der Nitrocellulosemembran mit auf 56°C erhitztem HRV2 durchgeführt. Diese Behandlung führt zu strukturellen Veränderungen des viralen Kapsids, die eine Bindung des Virus an der HeLa Zelloberfläche unmöglich macht (23). Wie in Fig. 5, Spalte 5 zu sehen, tritt unter diesen Bedingungen keine Bindung ein.

## Tabelle I

### Sensitivität des kleinen Rhinovirengruppenrezeptors

| Vorbehandlung des solubilisierten kleinen Gruppenrezeptors | Bindungsassay (% Bind.-akt. |
|---|---|
| keine Vorbehandlung | 100 |
| 10 µg Trypsin | 6 |
| 50 mU Neuraminidase | 170 |
| 10 mM Dithiothreitol | 15 |
| 10 mM Jodacetamid | 80 |
| 10 mM Natriumperiodat | 70 |
| 10 mM EDTA[a] | 5 |

Alle Vorinkubationen wurden bei 37° C für 30 min durchgeführt.

Beachte: (a) keine Vorbehandlung, stattdessen wurde die Inkubation mit markiertem HRV2 in Gegenwart von 10 mM EDTA durchgeführt.

## Tabelle II

**Kompetition verschiedener Rhinovirus-Serotypen für den kleinen Gruppenrezeptor.**

| Kompetition des radioaktiv markierten HRV2 mit : | Bindungsassay (% Bind.-akt.) |
|---|---|
| nichts | 100 |
| HRV2 | 13 |
| HRV89 | 95 |

| Kompetition des radioaktiv markiertem HRV49 mit: | |
|---|---|
| nichts | 100 |
| HRV2 | 15 |
| HRV89 | 90 |

Die Filter wurden wie beschrieben mit einem 20-fachem Überschuß mit nicht-markiertem Virus inkubiert.

Bibliographie

1. Abraham, G., and Colonno, R.J. (1984). Many rhinovirus serotypes share the same cellular receptor. J. Virol. 51, 340 - 345.
2. Blaas, D., Kuechler, E., Vriend, G., Arnold, E., Griffith, J.P., Luo, M., Rossmann, M.G. (1987). Comparison of three-dimensional structure of two human rhinoviruses (HRV2 and HRV14). Proteins (in press).
3. Brunette, D.M., and Till, J.E. (1971). A rapid method for the isolation of L-cell surface membranes using an aqueous two-phase polymer system. J. Membr. Biol. 5, 215 - 224.
4. Colonno, R.J., Callahan, P.L., and Long, W.J. (1986), Isolation of a monoclonal antibody that blocks attachment of the major group of human rhinoviruses. J. Virol. 57, 7 - 12.
5. Duechler, M., Skern, T., Sommergruber, W., Neubauer, Ch., Gruendler, P., Fogy, I., Blaas, D. and Kuechler, E. (1987). Evolutionary relationships within the human rhinovirus genus; comparison of serotypes 89, 2 and 14. Proc. Natl. Acad. Sci. U.S.A. (in press).
6. Fox, J.P. (1976). Is a rhinovirus vaccine possbible? American J.Epidemiol. 103, 345 - 354.
7. Krah, D.L., and Crowell, R.L. (1985). Properties of the desoxycholate-solubilized HeLa cell plasma membrane receptor for binding group B coxsackieviruses. J. Virol. 53: 867 - 870.
8. Lonberg-Holm, K., and Yin, F.H. (1973). Antigenic determinants of infective and inactivated human rhinovirus type 2. J. Virol. 12, 114 - 123.
9. Lonberg-Holm, K., and Whiteley, N.M. (1976). Physical and metabolic requirements for early interaction of poliovirus and human rhinovirus with HeLa cells. J. Virol. 19, 857 - 870.
10. Lonberg-Holm, K., Crowell, R.L., and Philipson, L. (1976). Unrelated animal viruses share receptors. Nature 259, 679 - 681.
11. Melnick, J.L. (1980). Taxonomy of Viruses. Proc. Med. Virol. 26, 214 - 232.

12. Noble-Harvey, J., and Lonberg-Holm, K. (1974). Sequential steps in attachment of human rhinovirus type 2 to HeLa cells. J. Gen. Virol. 25, 83 - 91.

13. Skern, T., Sommergruber, W., Blaas, D., Pieler, Ch., and Kuechler, E. (1984). The sequence of the polymerase gene of human rhinovirus type 2. Virology 136, 125 - 132.

14. Stott, E.J., and Heath, G.F. (1970). Factors affecting the growth of rhinovirus 2 in suspension cultures of L132 cells. J. gen. Virol. 6, 15 - 24.

15. Stott, E.J., Killington, R.A. (1972) Rhinoviruses. Ann. Rev. Microbiol. 26, 503 - 525.

16. Young, N.M., Leon, M.A., Takahashi, T., Howard, I.K., and Sage, H.J. (1971). Studies on a phytohemagglutinin from the lentil. III. Reaction of Lens culinaris hemagglutinin with polysaccharides, glycoproteins, and lymphocytes. J. Biol. Chem. 271, 1596 - 1601.

17. Tomassini, J.E. and Colonno, R.J. (1986). Isolation of a receptor protein involved in attachment of human rhinoviruses. J. Virol. 58, 290 - 295.

18. Köhler, G. and Milstein, C. (1975). Continuous cultures of fused cells secreting antibody of predefined specificity. Nature (London) 256, 495 -497.

19. Rossmann, M.G., Arnold, E., Erickson, J.W., Frankenberger, E.A., Griffith, J.P., Hecht, H-J., Johnson, J.E., Kamer, G., Luo, M., Mosser, A.M., Rueckert, R.R., Sherry, B.A, & Vriend, G. (1985). Structure of a common cold virus, human rhinovirus 14 (HRV14), Nature (London) 317, 145-154.

20. Mapoles, J.E., Krah, D.L. & Crowell, R.L. (1985). Purification of a HeLa cell receptor protein for group B coxsackieviruses. Journal of Virology 55, 560-566.

21. Laemmli, U.K. (1979). Cleavage of structural proteins during the assembly of the head of bacteriophage T4. Nature (London) 277, 680-685.

22. Burnette, W.N. (1981). Western blotting: electrophoretic transfer of proteins from sodium dodecyl sulfate - polyacrylamide gels to unmodified nitrocellulose and radiographic detection with antibody and radioiodinated protein A. Analytical Biochemistry 112, 195-203.

23. Lonberg-Holm, K. & Yin, F.H. (1973). Antigenic determinants of infective and inactivated human rhinovirus type 2. Journal of Virology 9, 29-40.

**Patentansprüche**

1. Rezeptor in wesentlicher reiner Form, dadurch gekennzeichnet, daß
   - eine Bindungsaktivität für Rhinoviren der kleinen Rezeptorgruppe vorhanden ist,
   - daß das Molekulargewicht, ermittelt durch Gelpermeationschromatographie, etwa 450 kD beträgt,
   - daß die Sedimentationskonstante, ermittelt durch Zuckergradientenzentrifugation in Gegenwart von Detergentien, etwa 28,4 S entspricht,
   - daß er von Lens culinaris Lectin gebunden wird,
   - daß er von Heparin-Sepharose nicht gebunden wird,
   - daß er irreversibel an einen Anionenaustauscher bindet,
   - daß seine Bindungsaktivität unempfindlich gegenüber Neuraminidase ist,
   - daß er aus Untereinheiten besteht, die durch intermolekulare Disulfidbrücken assoziiert sind,
   - daß er in Gegenwart von EDTA keine Bindung zu Rhinoviren aufweist,
   - daß Iodacetamid die Bindungsaktivität zu Rhinoviren nur wenig beeinflußt.

2. Rezeptoruntereinheit, dadurch gekennzeichnet, daß sie durch vollständige Reduktion aus dem Rezeptor nach Anspruch 1 herstellbar ist.

3. Rezeptor, dadurch gekennzeichnet, daß er aus mindestens zwei der Rezeptoruntereinheiten gemäß Anspruch 2 besteht und nicht der natürliche Rezeptor ist.

4. Rezeptor, herstellbar durch gezielte Reduktion des Rezeptors gemäß Anspruch 1.

5. Rezeptor, herstellbar aus einem Rezeptor gemäß Anspruch 1 durch gezielte Behandlung mit Enzymen und/oder Chemikalien, die nicht in einem der vorhergehenden Ansprüche bereits genannt sind.

6. Rezeptor, herstellbar durch gezielte Oxidation von mindestens zwei der Rezeptoruntereinheiten nach Anspruch 2.

**7.** Verfahren zur Herstellung von Rezeptoren mit Bindungsaktivität für Rhinoviren der kleinen Rezeptorgruppe gemäß Anspruch 1, dadurch gekennzeichnet, daß

a. Membranen aus Zellen, vorzugsweise HeLa-Zellen isoliert und gereinigt werden,

b. die Rezeptoren in den Membranen mit Detergentien, vorzugsweise Triton-X100, CHAPS, Zwittergent, Octylglucosid oder DOC, besonders bevorzugt Octylglucosid solubilisiert werden,

c. die unlöslichen Bestandteile entfernt werden und

d. die Rezeptoren durch Chromatographie, vorzugsweise an einer Concanavalin-A-, Ricin-Sepharose- oder Lens culinaris Lectinsäule gereinigt werden.

**8.** Verfahren nach Anspruch 7, dadurch gekennzeichnet, daß die Rezeptoren nach Behandlung mit Neuraminidase über eine Anionenaustauschersäule, vorzugsweise über eine Mono Q-Säule chromatographiert werden.

**9.** Verfahren nach einem der Ansprüche 7 oder 8, dadurch gekennzeichnet, daß anschließend an die

1. chromatographische Reinigung eine

2. chromatographische Reinigung an einer Superose Säule durchgeführt wird.

**10.** Verfahren zur Herstellung der Rezeptoruntereinheiten nach Anspruch 2, dadurch gekennzeichnet, daß der Rezeptor gemäß Anspruch 1 gezielt reduziert wird.

**11.** Verfahren zur Herstellung des Rezeptors nach Anspruch 3, dadurch gekennzeichnet, daß mindestens zwei der Rezeptoruntereinheiten nach Anspruch 2 gezielt oxidiert werden.

**12.** Verwendung der Rezeptoren nach einem der Ansprüche 1 bis 6

- zum qualitativen und/oder quantitativen Nachweis von Rhinoviren, wobei die Rezeptoren vorzugsweise an einem festen Träger gebunden sein können;

- zur analytischen Charakterisierung oder Reinigung von Rhinoviren;

- zur Herstellung von polyclonalen und/oder monoclonalen Antikörper;

- zur Herstellung pharmazeutischer Präparate.

**13.** Mittel zur therapeutischen Behandlung, dadurch gekennzeichnet, daß es neben pharmazeutisch inerten Hilfs- und/oder Trägerstoffen eine wirksame Menge eines Rezeptors nach einem der Ansprüche 1 bis 6 enthält.

**14.** Hybrid-Zellinie, dadurch gekennzeichnet, daß sie monoklonale Antikörper gegen einen der Rezeptoren nach einem der Ansprüche 1 bis 6 sezerniert.

**15.** Monoklonale Antikörper, dadurch gekennzeichnet, daß sie spezifisch die Rezeptoren nach einem der Ansprüche 1 bis 6 neutralisieren oder spezifisch an einen der besagten Rezeptoren binden.

**16.** Polyklonale Antikörper, dadurch gekennzeichnet, daß sie die Rezeptoren nach einem der Ansprüche 1 bis 6 neutralisieren oder an einen der besagten Rezeptoren binden.

**17.** Verwendung der polyklonalen und/oder monoklonalen Antikörper nach Anspruch 15 oder 16 zur qualitativen und/oder quantitativen Bestimmung eines der Rezeptoren nach einem der Ansprüche 1 bis 6 oder zur Reinigung eines der Rezeptoren nach einem der Ansprüche 1 bis 6.

**18.** Test Kit zur Bestimmung von Rhinoviren, dadurch gekennzeichnet, daß er Rezeptoren nach einem der Ansprüche 1 bis 6 enthält.

**19.** Verfahren zur Herstellung von monoklonalen Antikörpern nach Anspruch 15, dadurch gekennzeichnet, daß Wirtstiere mit einem der Rezeptoren nach einem der Ansprüche 1 bis 6 immunisiert, B-Lymphozyten dieser Wirtstiere mit Myelomzellen fusioniert, die die monoklonalen Antikörper ausscheidenden Hybrid-Zellinien subkloniert und in vitro oder in vivo kultiviert werden.

**Claims**

**1.** A receptor in substantially pure form, characterised in that

- it has binding activity for rhinoviruses of the small receptor group,
- the molecular weight, determined by gel permeation chromatography, is about 450 kD,
- the sedimentation constant, determined by sucrose gradient centrifugation in the presence of detergents, corresponds to about 28.4 S,
- it is bound by Lens culinaris lectin,
- it is not bound by heparin-Sepharose,
- it binds irreversibly to an anion exchanger,
- its binding activity is insensitive to neuraminidase,
- it consists of sub-units associated by intermolecular disulphide bridges,
- it shows no binding to rhinoviruses in the presence of EDTA,
- its binding activity to rhinoviruses is only slightly influenced by iodacetamide.

2. A receptor sub-unit, characterised in that it can be produced by complete reduction of a receptor as claimed in claim 1.

3. A receptor, characterised in that it consists of at least two of the receptor sub-units according to claim 2 and is not the natural receptor.

4. A receptor capable of being produced by controlled reduction of the receptor as claimed in claim 1.

5. A receptor capable of being prepared from a receptor as claimed in claim 1 by controlled treatment with enzymes and/or chemicals which have not already been specified in any of the preceding claims.

6. A receptor capable of being produced by controlled oxidation of at least two of the receptor sub-units as claimed in claim 2.

7. A process for preparing receptors with binding activity for rhinoviruses of the small receptor group according to claim 1, characterised in that
   a. membranes are isolated from cells, preferably HeLa cells, and purified,
   b. the receptors in the membranes are solubilised with detergents, preferably Triton-X100, CHAPS, Zwittergent, octyl glucoside or DOC, but particularly octyl glucoside, the insoluble constituents are removed and the receptors are purified by chromatography, preferably on a concanavalin-A, ricin-Sepharose or Lens culinaris lectin column.

8. A process as claimed in claim 7, characterised in that the receptors are chromatographed on an anion exchange column, preferably a Mono Q column, after treatment with neuraminidase.

9. A process as claimed in claim 7 or 8, characterised in that a second chromatographic purification is carried out on a Superose column after the first chromatographic purification.

10. A process for preparing the receptor sub-units as claimed in claim 2, characterised in that the receptor as claimed in claim 1 is reduced in controlled manner.

11. A process for preparing the receptor as claimed in claim 3, characterised in that at least two of the receptor sub-units as claimed in claim 2 are oxidised in controlled manner.

12. Use of the receptors according to one of claims 1 to 6
    - for the qualitative and/or quantitative detection of rhinoviruses, in which the receptors may preferably be bound to a solid carrier;
    - for the analytical characterisation or purification of rhinoviruses;
    - for the preparation of polyclonal and/or monoclonal antibodies;
    - for the production of pharmaceutical preparations.

13. Agent for therapeutic treatment, characterised in that it contains in addition to pharmaceutically inert excipients and/or carriers an effective amount of a receptor according to one of claims 1 to 6.

14. Hybrid cell line, characterised in that it secretes monoclonal antibodies against one of the receptors according to one of claims 1 to 6.

**15.** Monoclonal antibodies, characterised in that they specifically neutralise the receptors according to one of claims 1 to 6 or specifically bind to one of said receptors.

**16.** Polyclonal antibodies, characterised in that they neutralise the receptors according to one of claims 1 to 6 or bind to one of said receptors.

**17.** Use of the polyclonal and/or monoclonal antibodies according to claim 15 or 16 for the qualitative and/or quantitative determination of one of the receptors according to one of claims 1 to 6 or for purifying one of the receptors according to one of claims 1 to 6.

**18.** Test kit for determining rhinoviruses, characterised in that it contains receptors according to one of claims 1 to 6.

**19.** Process for preparing monoclonal antibodies according to claim 15, characterised in that host animals are immunised with one of the receptors according to one of claims 1 to 6, B-lymphocytes of these host animals are fused with myeloma cells, the hybrid cell lines secreting the monoclonal antibodies are sub-cloned and cultivated in vitro or in vivo.

## Revendications

**1.** Récepteur sous forme sensiblement pure, caractérisé en ce que:
- il existe une activité de liaison pour les rhinovirus du groupe récepteur mineur,
- le poids moléculaire, déterminé par chromatographie par perméation de gel, est d'environ 450 kd,
- la constante de sédimentation, déterminée par centrifugation en gradient de sucre en présence de détergents, correspond à environ 28,4 S,
- il est fixé par la lectine de Lens culinaris,
- il n'est pas fixé par l'héparine-Sepharose,
- il se lie de manière irréversible à un échangeur d'anions,
- son activité de liaison est insensible à la neuraminidase,
- il consiste en sous-unités qui sont associées par des ponts disulfure intermoléculaires,
- il ne présente aucune liaison aux rhinovirus en présence de EDTA,
- l'iodoacétamide n'a que peu d'influence sur l'activité de liaison aux rhinovirus.

**2.** Sous-unité de récepteur, caractérisée en ce qu'elle peut être obtenue par réduction totale à partir du récepteur selon la revendication 1.

**3.** Récepteur caractérisé en ce qu'il consiste en au moins deux des sous-unités de récepteur selon la revendication 2 et en ce qu'il n'est pas le récepteur naturel.

**4.** Récepteur qui peut être obtenu par réduction dirigée du récepteur selon la revendication 1.

**5.** Récepteur qui peut être obtenu à partir d'un récepteur selon la revendication 1 par traitement dirigé avec des enzymes et/ou des produits chimiques qui n'ont pas été déjà cités dans l'une des revendications précédentes.

**6.** Récepteur qui peut être obtenu par oxydation dirigée d'au moins deux des sous-unités de récepteur selon la revendication 2.

**7.** Procédé de préparation de récepteurs présentant une activité de liaison pour les rhinovirus du groupe récepteur mineur selon la revendication 1, caractérisé en ce que:
a. des membranes de cellules, de préférence de cellules HeLa, sont isolées et purifiées,
b. les récepteurs présents dans les membranes sont solubilisés avec des détergents, de préférence Triton-X100, CHAPS, Zwittergent, l'octylglucoside ou DOC, de préférence encore l'octylglucoside,
c. les constituants insolubles sont éliminés et
d. les récepteurs sont purifiés par chromatographie, de préférence sur une colonne de concanavaline A, de ricine-Sepharose ou de lectine de Lens culinaris.

**8.** Procédé selon la revendication 7, caractérisé en ce que les récepteurs sont chromatographiés sur une colonne échangeuse d'anions, de préférence sur une colonne de Mono Q, après le traitement avec la neuraminidase.

**9.** Procédé selon l'une des revendications 7 et 8, caractérisé en ce qu'à la suite de la première purification chromatographique on réalise une seconde purification chromatographique sur une colonne de Superose.

**10.** Procédé de préparation des sous-unités de récepteur selon la revendication 2, caractérisé en ce que le récepteur selon la revendication 1 est réduit de manière dirigée.

**11.** Procédé de préparation du récepteur selon la revendication 3, caractérisé en ce qu'au moins deux des sous-unités de récepteur selon la revendication 2 sont oxydées de manière dirigée.

**12.** Utilisation des récepteurs selon l'une des revendications 1 à 6
- pour la mise en évidence qualitative et/ou quantitative des rhinovirus, les récepteurs pouvant de préférence être liés à un support fixe;
- pour la caractérisation analytique ou la purification des rhinovirus;
- pour la préparation d'anticorps polyclonaux et/ou monoclonaux;
- pour l'obtention de préparations pharmaceutiques.

**13.** Agent de traitement thérapeutique, caractérisé en ce qu'il contient, outre des adjuvants et/ou véhicules inertes du point de vue pharmaceutique une quantité efficace d'un récepteur selon l'une des revendications 1 à 6.

**14.** Lignée cellulaire hybride, caractérisée en ce qu'elle sécrète des anticorps monoclonaux contre l'un des récepteurs selon l'une des revendications 1 à 6.

**15.** Anticorps monoclonaux, caractérisés en ce qu'ils neutralisent de manière spécifique les récepteurs selon l'une des revendications 1 à 6 ou se lient de manière spécifique à l'un desdits récepteurs.

**16.** Anticorps polyclonaux, caractérisés en ce qu'ils neutralisent les récepteurs selon l'une des revendications 1 à 6 ou se lient à l'un desdits récepteurs.

**17.** Utilisation des anticorps polyclonaux et/ou monoclonaux selon la revendication 15 ou 16 pour la détermination qualitative et/ou quantitative de l'un des récepteurs selon l'une des revendications 1 à 6 ou pour la purification de l'un des récepteurs selon l'une des revendications 1 à 6.

**18.** Trousse de test pour la détermination des rhinovirus, caractérisée en ce qu'elle contient des récepteurs selon l'une des revendications 1 à 6.

**19.** Procédé de préparation d'anticorps monoclonaux selon la revendication 15, caractérisé en ce que des animaux hôtes sont immunisés avec l'un des récepteurs selon l'une des revendications 1 à 6, des lymphocytes B de ces animaux hôtes sont fusionnés avec des cellules de myélome, les lignées cellulaires hybrides qui sécrètent les anticorps monoclonaux sont sous-clonées et cultivées in vitro ou in vivo.

FIG. 0

## FIG . 1

FIG. 2

FIG. 3